(19) Europäisches Patentamt
European Patent Office
Office européen des brevets

(11) **EP 2 659 957 A1**

(12) **EUROPEAN PATENT APPLICATION**
published in accordance with Art. 153(4) EPC

(43) Date of publication:
06.11.2013  Bulletin 2013/45

(51) Int Cl.:
*B01D 71/68* (2006.01)          *B01D 61/14* (2006.01)
*B01D 63/00* (2006.01)          *B01D 63/02* (2006.01)
*C12P 1/00* (2006.01)

(21) Application number: 11853437.9

(22) Date of filing: 22.12.2011

(86) International application number:
PCT/JP2011/079831

(87) International publication number:
WO 2012/090863 (05.07.2012 Gazette 2012/27)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority:  27.12.2010  JP 2010289638

(71) Applicant: Toray Industries, Inc.
Tokyo 103-8666 (JP)

(72) Inventors:
• KOBAYASHI, Atsushi
  Otsu-shi, Shiga 520-8558 (JP)
• CHEON, Jihoon
  Otsu-shi, Shiga 520-8558 (JP)
• TAKEUCHI, Norihiro
  Otsu-shi, Shiga 520-8558 (JP)
• NISHIDA, Makoto
  Otsu-shi, Shiga 520-8558 (JP)
• TANAKA, Yuji
  Otsu-shi, Shiga 520-8558 (JP)

(74) Representative: Hoefer & Partner
Pilgersheimer Straße 20
81543 München (DE)

(54)    **HOLLOW FIBER MEMBRANE MODULE AND METHOD FOR PRODUCING CHEMICALS**

(57)    Disclosed is a hollow fiber membrane module comprising a housing and a hollow fiber membrane filled in the housing, the space between the hollow fiber membrane and the housing being sealed with a sealing agent; wherein the hollow fiber membrane is made of a polysulfone based resin; a membrane surface average pore diameter is 5 nm or more and 500 nm or less; a pure water permeability coefficient under 50 kPa at 25°C is 0.05 $m^3/m^2$/hour or more and 5 $m^3/m^2$/hour or less; the sealing agent is at least one selected from a polyurethane resin and an epoxy resin; and a decrease in tensile strength after bringing the sealing agent into contact with saturated steam at 121°C for 24 hours is 25% or less. The present invention provides a hollow fiber membrane module which has sufficient durability against steam sterilization and maintains stable filtration property over a long period of time, and a method for producing a chemical substance by a continuous fermentation process, using the same.

Fig. 4

**Description**

[Technical Field]

The present invention relates to a hollow fiber membrane module, a method for producing a chemical substance by continuous fermentation.

[Background Art]

Fermentation processes, which are methods of substance production involving culturing of microorganisms or cultured cells, may be classified largely into: (1) batch and feed (fed-batch or semi-batch) fermentation processes; and (2) continuous fermentation processes. Batch and feed fermentation processes have simple facilities and terminate the culture in a short period of time, and in the case of fermentation of the product by culturing a pure strain, have an advantage of being less likely to cause contamination with microorganisms other than the microorganism being cultured, which is necessary for the culture. However, the concentration of the product in the fermentation broth increases with time, resulting in decrease in its productivity and yield due to inhibition by the product and increased osmotic pressure. Thus, it is difficult to stably maintain high yield and high productivity of the product over a long period of time.

In continuous fermentation processes, it is possible to maintain high yield and high productivity of a desired product over a long period of time, compared with the above-mentioned batch and feed fermentation processes, by avoiding the accumulation of the product in the fermenter. Conventional continuous culture processes are culture processes in which fresh medium is supplied at a specified rate into the fermenter and the cultured broth is withdrawn in the same volume outside the tank, so as to keep the liquid volume at a constant level at all times in the tank. Batch culture processes terminate the culture when the initial substrate has been consumed, whereas continuous culture processes theoretically allow the culture to be continued infinitely. That is, continuous culture processes theoretically make it possible for the fermentation to be carried out infinitely.

In conventional continuous culture processes, however, the microorganisms are also withdrawn along with the cultured broth outside the tank, and thus it is difficult to maintain microorganisms at a high concentration in the fermenter. In the case of production by fermentation, if the microorganism by which the fermentation is performed is allowed to be kept at a high concentration, then it is possible to improve the efficiency of fermentation production per fermentation volume. In order to keep the microbial concentration at a high level in the fermenter, it is necessary to retain or circulate microorganisms in the tank.

Thus, there have been proposed, in continuous culture processes, methods in which the microorganisms or cultured cells are separated or filtered, with separation membranes and the product is collected from the filtrate, and at the same time, the separated microorganisms or cultured cells are retained or circulated in the fermentation broth, whereby the concentration of the microorganisms or cultured cells is maintained at a high level in the fermentation broth.

Patent Literature 1 discloses a method for the production of succinic acid using a separation membrane. This technology adopts the use not only of ceramic membranes, but also of organic membranes, resulting in an extension of the range and type of membranes that can be applied to continuous fermentation technology. In the disclosed technology, however, a high filtration pressure (about 200 kPa) and a high linear velocity on membrane surface (2 m/second) were employed in the membrane separation. Such a high filtration pressure and a high linear velocity not only are disadvantageous in terms of costs, but also lead to physical damage to the microorganisms or cultured cells due to high pressure and shearing force in filtration processing. Furthermore, increased filtration pressure and linear velocity on membrane surface will become situations where a higher friction force is caused between the fluid and the membrane, resulting in a higher pressure loss. These make it difficult to maintain the operating conditions, and therefore, the disclosed technology is not suitable in continuous fermentation processes in which the microorganism or cultured cells are continuously returned into the fermentation broth.

In addition, membrane modules used in continuous fermentation processes are required to be sterilized before used. Sterilization methods include steam sterilization, dry-heat sterilization, gamma-ray sterilization, and the like. In industrial processes, steam sterilization would be most effective in sterilization of a fermenter and a membrane module in their connected state. For steam sterilization, the membrane module is required to have a sufficient level of wet and heat resistance. For example, Patent Literature 2 proposes that use should be made of bundling members which have a low rate of decrease in hardness after contact with high-temperature steam.

Patent Literature 3 proposes a method in which a separation membrane is applied in a fermentation process, which is a process for substance production involving culturing of microorganisms, to perform continuous fermentation, and the microorganisms or cultured cells are accumulated, so that the rate of production of the product is increased. In this technology, a fermenter is first provided, a membrane separation tank into which a flat membrane and a hollow-fiber membrane are placed is provided, a pump is used to transfer a fermentation broth from the fermenter into the membrane separation tank, and the filtration is controlled using a controller of the water head difference that is further provided separately from the membrane unit of the membrane separation tank. Since the two tanks and the controller are provided, a large area for their placement is required. In this technology, problems are posed in terms of the placement and maintenance/management of the facilities, such as necessity of disconnecting and stopping the membrane separation tank for the exchange of the separation membrane, resulting in a decreased rate of production of the product. In addition, there is a problem that a stable continuous operation over a long period of time cannot be achieved when continuous fermentation operations are carried out using the separation membrane disclosed in Patent Literature 3.

[Citation List]

[Patent Literature]

[Patent Literature 1] JP 2005-333886 A
[Patent Literature 2] WO 2011-058983 A
[Patent Literature 3] JP 2008-237213 A

[Summary of Invention]

[Technical Problem]

An object of the present invention is to provide a hollow fiber membrane module which has sufficient durability against steam sterilization and maintains stable filtration property over a long period of time, and a method for producing a chemical substance by a continuous fermentation process, using the same

[Solution to Problem]

In order to achieve the above object, the present invention includes the following configurations.

(1) A hollow fiber membrane module comprising a housing and a hollow fiber membrane filled in the housing, the space between the hollow fiber membrane and the housing being sealed with a sealing agent; wherein the hollow fiber membrane is made of a polysulfone based resin; a membrane surface average pore diameter is 5 nm or more and 500 nm or less; a pure water permeability coefficient under 50 kPa at 25°C is 0.05 $m^3/m^2$/hour or more and 5 $m^3/m^2$/hour or less; the sealing agent is at least one selected from a polyurethane resin and an epoxy resin; and a decrease in tensile strength after bringing the sealing agent into contact with saturated steam at 121°C for 24 hours is 25% or less.
(2) A method for producing a chemical substance, which comprises the steps of using a fermentation broth containing a fermentation raw material, a chemical substance, and microorganisms or cultured cells, filtering the fermentation broth, filtering the fermentation broth through a separation membrane module, collecting the chemical substance from the filtrate, and at the same time retaining or circulating the unfiltered liquid in the fermentation broth; and adding the fermentation raw material to the fermentation broth; the chemical substance being produced by continuous fermentation; wherein the hollow fiber membrane module according to (1) is used as the separation membrane module.

[Advantageous Effects of Invention]

The hollow fiber membrane module of the present invention has sufficient durability against steam sterilization. The hollow fiber membrane module of the present invention enables the production, which can stably maintain high productivity over a long period of time, by continuous fermentation, and thus making it possible to stably produce a chemical substance, which is a fermentation product, at low cost in a wide range of fermentation industry.

[Brief Description of the Drawings]

Fig. 1 is a schematic longitudinal cross-sectional view illustrating an example of a hollow fiber membrane module of the present invention.
Fig. 2 is a schematic longitudinal cross-sectional view illustrating an example of a hollow fiber membrane module of the present invention.

Fig. 3 is a schematic longitudinal cross-sectional view illustrating an example of a hollow fiber membrane module of the present invention.

Fig. 4 is a schematic view illustrating an example of a membrane separation apparatus for continuous fermentation used in the present invention.

[Description of Embodiments]

The hollow fiber membrane module of the present invention is a hollow fiber membrane module in which a hollow fiber membrane is filled in a housing and the space between the hollow fiber membrane and the housing is sealed with a sealing agent. As used herein, the phrase "the space between the hollow fiber membrane and the housing is sealed with a sealing agent" refers to the fact that the hollow fiber membrane and the housing are fluid-tightly fixed to each other through a sealing agent. The hollow fiber membrane module may includes an aspect in which the hollow fiber membrane and the housing may be directly bonded to each other by a sealing agent or, as described in detail below, the hollow fiber membrane may be bonded to a tubular case by a sealing agent, and the tubular case may be fluid-tightly fixed to the housing by a gasket. In order to perform steam sterilization, it is preferred that the hollow fiber membrane and the housing are fluid-tightly and air-tightly fixed to each other through a sealing agent.

In the hollow fiber membrane module of the present invention, a hollow fiber membrane is used as a separation membrane. The hollow fiber membrane is advantageous in that, when employing a form (module) used actually in filtration, it is possible to increase the filling membrane area per unit volume, leading to an increase in throughput per volume, compared with a flat membrane or a sheet-like membrane.

The hollow fiber membrane can be produced by using, as a raw liquid for membrane production, a solution containing a polymer, additives and a solvent as main components; and discharging the raw liquid to a solidification bath from a spinneret while injecting a hollow section-forming fluid in the case spinning. In the present invention, a polysulfone based resin is used as the polymer. Since the polysulfone based resin exhibits excellent adhesion to both the polyurethane resin and the epoxy resin used as the sealing agent, durability against steam sterilization is improved. Specific examples of the polysulfone based resin include polysulfone, polyethersulfone, poly (aryl ether sulfone) and polyallylate-polyethersulfone, and polysulfone or polyethersulfone is preferably used. Use of the aromatic polysulfone is more preferred since the strength or heat resistance of the hollow fiber membrane is improved.

Examples of preferable additive include tetraethylene glycol, ethylene glycol, triethylene glycol, nitrobenzene, tetrahydrofuran, dioxane, dimethyl carbonate, diethyl phosphate, polyvinylpyrrolidone, cellulose derivatives and the like.

The solvent may dissolve both the polymer and the additives. Examples of preferable solvent include dimethyl sulfoxide, N-methyl-2-pyrrolidone, dimethylacetamide and the like.

The concentration of the polymer in the raw liquid for membrane production may be in any range as long as the membrane can be produced and the obtained membrane has a property as a membrane. Usually, the concentration of the polymer is preferably from 10 to 30% by weight. In order to obtain a membrane having high water permeability, the concentration of the polymer is preferably decreased and, more preferably, the concentration of the polymer is adjusted within a range from 10 to 20% by weight.

The concentration of additives in the polymer solution varies depending on the type and molecular weight of additives, and is preferably adjusted within a range from 1 to 30% by weight, and more preferably from 1 to 25% by weight. For the purpose of controlling the viscosity and dissolved state of a raw liquid for membrane production, it is also possible to add components such as water and salt. The type and addition amount may be appropriately selected by combination.

The pore diameter of the hollow fiber membrane is preferably 5 nm or more and 500 nm or less in terms of a membrane surface average pore diameter. More preferably, the membrane surface average pore diameter is 10 nm or more and 200 nm or less. The membrane surface average pore diameter is not preferably less than 5 nm since a decrease in filtration flow rate may occur. In contrast, the membrane surface average pore diameter is not preferably more than 500 nm since it may become impossible to perform effective filtration fractionation of turbidity, and also turbidity may cause clogging inside the membrane, leading to significant decrease in filtration amount with time.

The membrane surface average pore diameter can be determined in accordance with the method defined in ASTM: F316-86 (another name: half dry method). Determined by this half dry method is an average pore diameter of a minimum pore diameter layer of the membrane. In the present invention, the measurement of the membrane surface average pore diameter by the half dry method is performed under standard measurement conditions at 25°C and a pressure increase rate of 0.001 MPa/second, using ethanol as the liquid. The membrane surface average pore diameter [μm] is determined by the following equation.

Membrane surface average pore diameter [μm] = (2,860 × surface tension [mN/m])/half dry air pressure [Pa], where the surface tension is a surface tension of a liquid to be used, namely, ethanol. Since the surface tension of ethanol at 25°C is 21.97 mN/m (Kagaku Binran Kisohen (Handbook of Chemistry, Basic Course), Revised edition 3, p.

II-82, published by Maruzen Co., Ltd., edited by the Chemical Society of Japan, 1984)), it is possible to determine the membrane surface average pore diameter by the following equation:

$$\text{Membrane surface average pore diameter } [\mu m] = 62834.2/(\text{half dry air pressure } [Pa]).$$

The hollow fiber membrane preferably has a pure water permeability coefficient under 50 kPa at 25°C of 0.05 $m^3/m^2$/hour or more and 5 $m^3/m^2$/hour or less from the viewpoint of durability against tension, rapture or compression, and permeation property. The pure water permeability coefficient can be measured by the following method.

After producing a hollow fiber membrane module, the hollow fiber membrane module was immersed in ethanol to fill inside pores of the hollow fiber membrane with ethanol, and then ethanol was replaced by water by repeating immersion in pure water several times. Thereafter, pure water at 25°C was supplied into the module under a pressure of 50 kPa and the permeation water amount of pure water permeated through the membrane was measured, and then a pure water permeability coefficient was determined by the following equation:

Pure water permeability coefficient $[m^3/m^2/\text{hour}]$ = water permeability $[m^3]/(\pi \times$ membrane outer diameter $[m]$ $\times$ membrane effective length $[m/\text{sheet}] \times$ number of membranes $[\text{sheets}] \times$ measurement time $[\text{hour}]$), where the membrane effective length refers to a net membrane length, excluding the portion bonded with a sealing agent.

It is also preferred to use, as needed, a hydrophilized membrane in which 0.1% by weight or more and 10% by weight or less of an ethylene-vinyl alcohol copolymer is retained on a surface of a polysulfone based resin hollow fiber membrane. As used herein, the phrase "ethylene-vinyl alcohol copolymer is retained on surface of a polysulfone based resin hollow fiber membrane" means that at least a part of a surface of a polysulfone based resin hollow fiber membrane is coated with an ethylene-vinyl alcohol copolymer.

The ethylene-vinyl alcohol copolymer may be any type of copolymers, such as a random copolymer, a block copolymer and a graft copolymer. The ethylene content in the copolymer is preferably within a range of 20 mol% or more and 60 mol% or less. The ethylene content of less than 20 mol% is not preferred because of poor adhesion to a polysulfone resin, while the ethylene content of more than 60 mol% is not preferred because of disappearance of hydrophilicity. The ethylene-vinyl alcohol copolymer is, for example, a crystalline thermoplastic resin which is synthesized by copolymerizing ethylene with vinyl acetate, and saponifying (hydrolyzing) an acetic acid ester moiety of a vinyl acetate-derived side chain to convert the side chain into a hydroxyl group.

The amount of the ethylene-vinyl alcohol copolymer retained in the hollow fiber membrane is preferably 0.1% by weight or more from the viewpoint of the effect of fouling resistance on an organic substance, and preferably 10% by weight or less from the viewpoint of water permeability. The retention amount is more preferably 0.5% by weight or more and 7% by weight or less, and still more preferably 1% by weight or more and 5% by weight or less. The retention amount of less than 0.1% by weight may lead to less effect of hydrophilicity, whereas, the retention amount of more than 10% by weight may lead to a decrease in water permeability of the membrane.

The retention amount of the ethylene-vinyl alcohol copolymer was determined by the following equation:

$$\text{Retention amount (\% by weight)} = 100 \times \{(\text{dry membrane weight } [g] \text{ of ethylene-vinyl alcohol copolymer coated polysulfone based resin membrane}) - (\text{dry membrane weight } [g] \text{ of polysulfone based resin membrane})\}/(\text{dry membrane weight } [g] \text{ of ethylene-vinyl alcohol copolymer coated polysulfone based resin membrane}), \text{ where}$$

a dry membrane was obtained by drying in an oven at 60°C.

The method of retaining an ethylene-vinyl alcohol copolymer on a surface of a polysulfone based resin hollow fiber membrane includes, for example, a method in which an ethylene-vinyl alcohol copolymer is dissolved in a mixed solution of an alcohol such as methanol, ethanol or 2-propanol, and water, and a polysulfone based resin hollow fiber membrane is immersed in the solution, followed by drying.

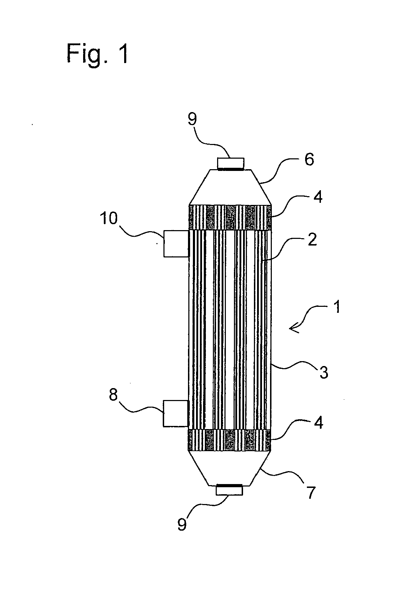

Next, the configuration of a hollow fiber membrane module of the present invention will be described with reference to the accompanying drawings. Fig. 1 is a schematic longitudinal cross-sectional view illustrating an example of a hollow fiber membrane module of the present invention. A hollow fiber membrane module 1 illustrated in Fig. 1 has the configuration in which a large number of hollow fiber membranes 2 are accommodated in a housing 3, both ends of which are opened, and both ends of each of hollow fiber membrane bundles are fluid-tightly and air-tightly fixed to the housing 3 by a sealing agent in a state where at least one of end surfaces of the hollow fiber membrane 2 is opened (hollow fiber membrane sealing member 4). An upper cap 6 having a filtrate outlet 9 and a lower cap 7 having a filtrate outlet 9 are fluid-tightly and air-tightly connected with the upper part and the lower part of the housing 3, respectively. A fermentation broth flows into the module through a fermentation broth inlet 8, while a fermentation broth not permeating through the hollow fiber membrane 2 is discharged from a fermentation broth outlet 10 outside of the hollow fiber membrane module 1. The filtrate permeated through the hollow fiber membrane 2 is discharged from the upper and lower filtrate outlets 9 outside of the hollow fiber membrane module 1. The filtrate can also be discharged from any one of the upper and lower filtrate outlets 9.

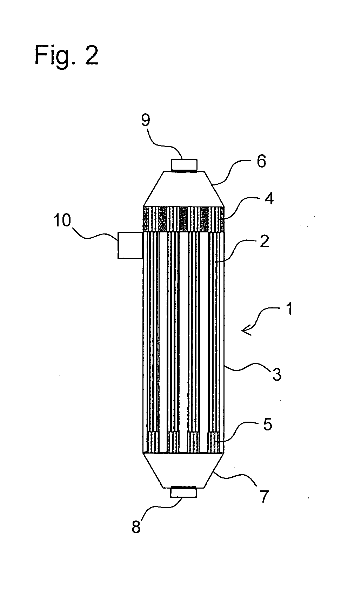

Fig. 2 is a schematic longitudinal cross-sectional view illustrating the other one example of a hollow fiber membrane module of the present invention. A hollow fiber membrane module 1 illustrated in Fig. 2 has the configuration in which a large number of hollow fiber membranes 2 are accommodated in a housing 3, both ends of which are opened, and are fluid-tightly and air-tightly fixed to the housing 3 by a sealing agent in a state where the end surface of the upper part of the hollow fiber membrane 2 is opened. At the end surface of the lower part of the hollow fiber membrane 2, plural hollow fiber membranes are bundled to form a small bundle sealing member 5 in which the hollow section of the hollow fiber membrane is sealed with a sealing agent. An upper cap 6 having a filtrate outlet 9 and a lower cap 7 having a fermentation broth inlet 8 are fluid-tightly and air-tightly connected with the upper part and the lower part of the housing 3, respectively. A fermentation broth not permeating through the hollow fiber membrane 2 is discharged from a fermentation broth outlet 10 outside of the hollow fiber membrane module 1. The filtrate permeated through the hollow fiber membrane 2 is discharged from the filtrate outlets 9 outside of the hollow fiber membrane module 1.

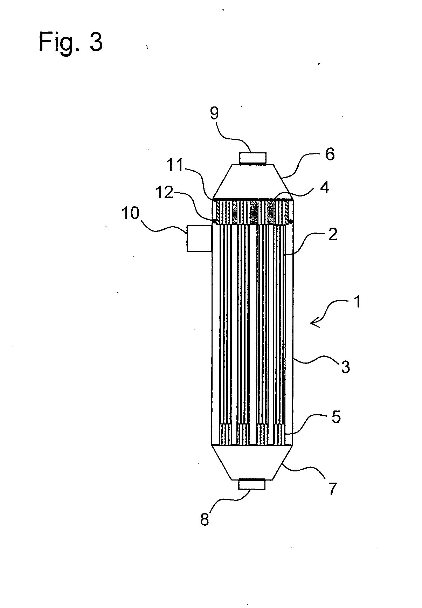

Fig. 3 is a schematic longitudinal cross-sectional view illustrating the other one example of a hollow fiber membrane module of the present invention. A hollow fiber membrane module illustrated in Fig. 3 resembles the hollow fiber membrane module illustrated in Fig. 2, but differs in that it constitutes a cartridge in which the hollow fiber membranes 2 are not directly fixed to the housing 3 and the hollow fiber membranes 2 are filled in a tubular case 11. The space between the hollow fiber membrane 2 and the tubular case 11 is fluid-tightly and air-tightly fixed by a sealing agent. The space between the tubular case 11 and the housing 3 is fluid-tightly and air-tightly fixed by an O-ring 12. It is also possible to preferably use such cartridge type hollow fiber membrane module.

In the present invention, at least one selected from a polyurethane resin and an epoxy resin can be used as the sealing agent of the hollow fiber membrane module. It is necessary for the sealing agent that a decrease in tensile strength after bringing the sealing agent into contact with saturated steam at 121°C for 24 hours is 25% or less, so as to have sufficient durability against steam sterilization. The decrease in tensile strength is more preferably 20% or less. The decrease in tensile strength is determined as follows: that is, a specimen is produced using a resin used in a sealing agent and the specimen is treated with steam at 121°C for 24 hours, followed by the measurement of the tensile strength of the specimen before and after the treatment in accordance with JIS K 6251 (2004). Details will be mentioned below.

In the production of a chemical substance by a continuous fermentation process, a hollow fiber membrane module is used after subjecting to steam sterilization in advance. When using a sealing agent in which a decrease in tensile strength after bringing the sealing agent into contact with saturated steam at 121°C for 24 hours is more than 25%, the sealing agent may deteriorate upon steam sterilization to cause a decrease in adhesive strength, and thus being likely to cause peeling between the sealing agent and the housing, leading to the generation of bacterial contamination from the peeled portion.

When peeling between the sealing agent and the housing occurs upon steam sterilization, bacterial contamination may occur from the peeled portion. The hollow fiber membrane made of a polysulfone based resin used in the present invention exhibits excellent adhesion to both a polyurethane resin and an epoxy resin, and is less likely to cause peeling even when subjected to steam sterilization over a long period of time.

The polyurethane resin can be obtained by reacting an isocyanate with a polyol. There is no particular limitation on the type of the isocyanate as long as the reaction product has sufficient moist heat resistance, and examples thereof include tolylene diisocyanate (TDI), diphenylmethane diisocyanate(MDI), polymethylene polyphenyl isocyanate (Polymeric MDI.), xylylenediisocyanate (XDI) and the like. These isocyanates may be used alone, or two or more isocyanates may be used in combination.

There is no particular limitation on the type of the polyol as long as the reaction product has sufficient moist heat resistance, and examples thereof include a polybutadiene based polyol, a dimer acid modified polyol, an epoxy resin modified polyol, a polytetramethylene glycol and the like. These polyols maybe used alone, or two or more polyols maybe used in combination. As long as moist heat resistance of the reaction product is not impaired, other types of polyols, for example, castor oil based polyol, polycarbonatediol, polyesterpolyol and the like can be used in combination.

The epoxy resin can be obtained by reacting a main component with a curing agent. There is no particular limitation on the type of the main component as long as the reaction product has sufficient moist heat resistance, and examples thereof include a bisphenol type epoxy resin, a novolak type epoxy resin, a naphthalene type epoxy resin, a cyclopentadiene type epoxy resin and the like. There is no particular limitation on the type of the curing agent as long as the reaction product has sufficient moist heat resistance, and examples thereof include an aliphatic amine, an aromatic amine, an organic acid anhydride based or modified amine and the like. Among these curing agents, an aliphatic polyamine can be preferably used.

The addition of a filler to the sealing agent enables suppression of heat generated by the reaction, and thus making it possible to reduce shrinkage stress and to inhibit the generation of cracking of the reaction product and peeling from the housing, and to enhance the strength.

Silica, calcium carbonate and a glass fiber can be used as the filler, and silica is preferably used. The additive amount of the filler in a sealing agent resin is preferably from 1% by weight to 60% by weight of the sealing agent resin.

There is no particular limitation on raw materials of the housing and tubular case used in the hollow fiber membrane module of the present invention, as long as they can be subjected to steam sterilization, and examples thereof include fluorine based resins such as polysulfone based resin, polytetrafluoroethylene and perfluoroalkoxyfluororesin; polycarbonate, polypropylene, polymethylpentene, polyphenylene sulfide, polyether ketone, stainless steel and the like.

In the case of a cartridge type hollow fiber membrane module, a tubular case and a housing are fluid-tightly fixed to each other by a gasket. In order to perform steam sterilization, it is preferred that the tubular case and the housing are fluid-tightly and air-tightly fixed to each other. As the gasket, for example, an O-ring can be used. The material of the gasket may be a material which is less likely to undergo deterioration by steam sterilization, and a material having strong durability against an acid, an alkali and chlorine is more preferably used. Examples of the material include a fluororubber, a silicone rubber, an ethylene-propylene-diene rubber (EPDM) and the like.

The method for producing a chemical substance of the present invention will be described below. The method for producing a chemical substance of the present invention is a method for producing a chemical substance, which comprises the steps of using a fermentation broth containing a fermentation raw material, a chemical substance, and microorganisms or cultured cells, filtering the fermentation broth, filtering the fermentation broth through a separation membrane module, collecting the chemical substance from the filtrate, and at the same time retaining or circulating the unfiltered liquid in the fermentation broth; and adding the fermentation raw material to the fermentation broth; the chemical substance being produced by continuous fermentation. The above hollow fiber membrane module is used as the separation membrane module.

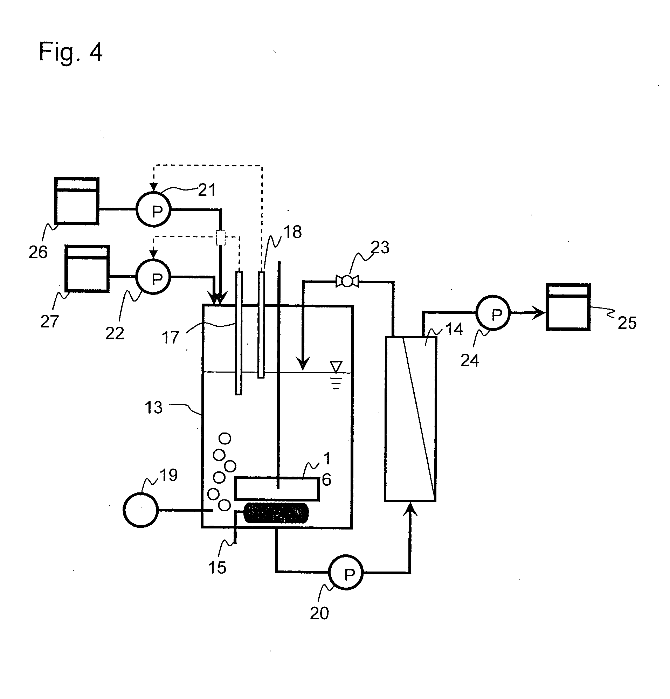

Fig. 4 is a schematic view illustrating an example of a membrane separation apparatus for continuous fermentation used in the present invention. In Fig. 4, a continuous fermenter is basically composed of a fermenter 13, a separation membrane module 14, a filtrate reservoir 25, and a fermentation raw material tank 26 and a neutralizer tank 27.

In Fig. 4, in the fermenter 13, a fermentation raw material supply pump 21 is controlled by a level sensor/controller 18 and the fermentation raw material is charged in the fermenter 13. As needed, a fermentation broth in the fermenter 13 is stirred by a stirrer 16. As needed, the temperature of the fermentation broth is controlled by a temperature controller 15. As needed, a required gas can be supplied to the fermentation broth by a fermenter gas supply device 19. It is also possible to supply the supplied gas again by the fermenter gas supply device 19 after collecting and recycling. As needed, it is also possible to perform fermentation production with high productivity by controlling a neutralizer supply pump 22 and adjusting the pH of the fermentation broth using a pH sensor/controller 17.

The fermentation broth in the fermenter 13 contains a fermentation raw material, a chemical substance obtained by fermentation, and microorganisms or cultured cells for performing fermentation. This fermentation broth is supplied from the fermenter 13 to the separation membrane module 14 by a circulation pump 20. The fermentation broth is filtered and separated into microorganisms or cultured cells, and a filtrate containing a chemical substance by a separation membrane module 14. The filtrate containing a chemical substance is taken out from an apparatus and transferred to a filtrate reservoir 25. The hollow fiber membrane module of the present invention is used as the separation membrane module 14. The thus filtered and separated microorganisms or cultured cells are circulated in the fermenter 13. Whereby, the concentration of microorganisms in the fermenter 13 can be maintained at a high level, and thus enabling fermentation production at a high production rate. The filtration step by the separation membrane module 14 can be carried out by a pressure due to the circulation pump 20 without using a special power and, as needed, the amount of the fermentation broth can be moderately adjusted by providing a filtration pump 24. As needed, it is also possible to adjust the amount of the fermentation broth by providing a circulation return control valve 23 and increasing a pressure applied to the separation membrane module 14.

The above-mentioned membrane separation filtration step makes it possible to provide a method for producing a chemical substance by a continuous fermentation process, which stably maintains high productivity over a long period of time.

In the present invention, the membrane filtration method may be either a dead end filtration or cross flow filtration method. However, in the case of the continuous fermentation operation, since membrane contamination substances such as microorganisms and membrane clogging substances adheres to the membrane, it is preferred to perform filtration while removing the membrane contamination substances by a shear force of fermentation broth flow due to cross flow. It is effective for the removal of the membrane contamination substances when air scrubbing washing is performed together with cross flow filtration.

The fermenter is preferably made of materials which are superior in pressure resistance, heat resistance and fouling resistance. The shape of fermenter may be any shape in which a fermentation raw material, a microorganism, and other solid(s), liquid(s), and gas (es) necessary for fermentation can be placed and mixed, and which can be sterilized as needed, and be tightly closed, including cylindrical, polygonal tube-typed, and other shapes. The shape is preferably cylindrical, when considering the efficiency of mixing of a fermentation raw material and a culture medium and a microorganism, and other factors. The fermenter is preferably equipped with a pressure gauge, so that the fermenter is maintained at all times in pressurized conditions where the pressure in the fermenter is pressurized, in order to prevent microorganisms from entering into the inside from the outside of the fermenter and growing in the fermenter.

A fermentation raw material which is used in the present invention may be any one which can promote the growth of a microorganism or cultured cells which are cultured for the fermentation and allow satisfactory production of a chemical substance that is a desired fermentation product. As a fermentation raw material, for example, a liquid medium or the like is preferably used in which a carbon source, a nitrogen source, inorganic salts, and optionally amino acids and organic trace nutrients such as vitamins are appropriately contained.

In the case of a liquid containing some materials which can promote the growth of a microorganism or cultured cells which are cultured for the fermentation and allow satisfactory production of a chemical substance that is an desired fermentation product, for example, wastewater or sewage water may be also used directly, or with sterilization treatment or the like, or with addition of fermentation raw materials.

As the above-mentioned carbon source, use is made of, for example, sugars such as glucose, sucrose, fructose, galactose and lactose; starch, starch hydrolysates, sweet potato molasses, sugar beet molasses, cane juice containing these sugars; extracts or concentrates from sugar beet molasses or cane juice; filtrates, syrups (high test molasses) of sugar beet molasses or cane juice; raw sugars purified or crystallized from sugar beet molasses or cane juice; white sugars purified or crystallized from sugar beet molasses or cane juice; and additionally organic acids such as acetic acid and fumaric acid, alcohols such as ethanol and glycerin. Sugars are the first oxidation product of polyhydric alcohols and refer to carbohydrates which have an aldehyde group or a ketone group and in which sugars having an aldehyde group are classified as aldose and sugars having a ketone group as ketose.

As the above-mentioned nitrogen source, use is made of, for example, ammonia gas, aqueous ammonia, ammonium salts, urea, nitrates, and besides these, organic nitrogen sources which are supplementally used, such as oil cake, soy bean hydrolysates, casein hydrolysates, other amino acids, vitamins, corn steep liquor, yeast or yeast extracts, meat extracts, peptides such as peptone, various fermented microbial cells and hydrolysates thereof.

It is possible to appropriately use, as the above-mentioned inorganic salts, for example, phosphates, magnesium salts, calcium salts, iron salts, manganese salts, and the like.

To a fermentation raw material are preferably added nutrients necessary for microbial growth, so that continuous microbial growth is achieved. The concentration of the microorganism or cultured cells in the culture medium is preferably maintained at a high level in the range which does not result in an increased ratio of their death due to the culture medium environment being inappropriate for their growth, in order to achieve high productivity of the product. The details of microorganisms or cultured cells in the culture medium are mentioned below in conjunction with chemical substances obtained by the production method of the present invention.

Continuous fermentation operations, which are performed while allowing proliferation of fresh microbial cells capable of fermentation production, are preferably carried out in a single fermenter in usual cases, in terms of culture control. In the case of continuous fermentation culture processes which allow production of the product with proliferation of the microbial cells, however, there are no limitations on the number of fermenters. A plurality of fermenters may be used for reasons, such as their small capacities. In this case, the high productivity of the fermentation product will be obtained even when the fermenters are connected by piping in parallel or in tandem to perform continuous culture.

Fermentation culture conditions are appropriately set, depending upon the kind of microorganism or cultured cells, and the desired product, and generally are at a pH of 3 or more and 10 or less and at a temperature of 15°C or higher and 65°C or lower in many cases. The pH of the culture medium is adjusted to a determined value using an inorganic or organic acid, an alkaline substance, and additionally urea, calcium hydroxide, calcium carbonate, ammonia gas, and the like.

In the method for producing a chemical substance according to the present invention, continuous culture may be started after batch or fed-batch culture is performed at an early stage of the culture to increase the concentration of the microorganism or cultured cells. In addition, continuous culture may be performed with the starting of the culture by increasing the microbial concentration, followed by seeding of microbial cells of the increased concentration. It is also possible to supply a fermentation raw material and to withdraw the cultured medium from appropriate points of time. The points of time at which the supply of a fermentation raw material and the withdrawing of the cultured medium are carried out are not necessarily the same. The supply of a fermentation raw material and the withdrawing of the cultured medium may be carried out in a continuous or intermittent manner.

The chemical substance which is obtained by the production method of the present invention is one which the above-mentioned microorganism or cultured cells produce into the culture medium. Examples of such a chemical substance may include, for example, substances produced by mass production in the fermentation industry, such as alcohols, organic acids, amino acids, and nucleic acids. The present invention can be also applied to the production of substances like enzymes, antibiotics, and recombinant proteins. For example, the alcohols may include ethanol, 1,3-butanediol, 1,4-butanediol and glycerol. The organic acids may include acetic acid, lactic acid, pyruvic acid, succinic acid, malic acid, itaconic acid, amino acids and citric acid. The nucleic acids may include inosine, guanosine and cytidine.

The amino acids include L-threonine, L-lysine, L-glutamic acid, L-tryptophan, L-isoleucine, L-glutamine, L-arginine, L-alanine, L-histidine, L-proline, L-phenylalanine, L-aspartic acid, L-tyrosine, L-methionine, L-serine, L-valine, and L-leucine, with L-threonine, L-lysine, and L-glutamic acid is particularly preferable among them.

As microorganism or cultured cells which are used in the present invention, eukaryotic or prokaryotic cells are employed. For example, there are included bacteria, such as *Escherichia coli*, lactobacilli, coryneform bacteria, and actinomycetes, yeasts, filamentous fungi, animal cells, and insect cells, which are often used in the fermentation industry. Microorganism or cultured cells which are used may be ones which have been isolated from the natural environment or modified in some properties by mutagenesis or genetic recombination.

Regarding microorganisms which are used in the present invention, for example, microorganisms which are capable of efficient production of L-amino acids can be preferably utilized, since the present invention consists in making fermentation culture more efficient. Such microorganisms include bacteria, such as *Escherichia coli* and coryneform bacteria, which are often used in the fermentation industry. Examples of strains producing L-amino acids are as indicated below.

L-Threonine producing strains include, for example, bacteria belonging to the genus *Escherichia,* the genus *Providencia,* the genus *Corynebacterium,* the genus *Brevibacterium*, or the genus *Serratia.* Among these bacteria, particularly preferable species are *Escherichia coli, Providencia rettgeri, Corynebacterium glutamicum, Brevibacterium flavum, Brevibacterium lactofermentum,* and *Serratia marcescens.*

L-Lysine producing strains include, for example, bacteria belonging to the genus *Escherichia,* the genus *Corynebacterium,* or the genus *Brevibacterium.* Among these bacteria, particularly preferable bacteria are *Escherichia coli, Corynebacterium glutamicum, Brevibacterium flavum,* and *Brevibacterium lactofermentum.*

L-Glutamic acid producing strains preferably are *Corynebacterium glutamicum, Brevibacterium flavum,* and *Brevibacterium lactofermentum.*

L-Tryptophan producing strains include, for example, *Corynebacterium glutamicum, Brevibacterium flavum, Brevibacterium lactofermentum, Bacillus subtilis, Bacillus amyloliquefaciens,* and *Escherichia coli.*

L-Isoleucine producing strains include, for example, *Corynebacterium glutamicum, Brevibacterium flavum, Brevibacterium lactofermentum,* and *Serratia marcescens.*

L-Glutamine producing strains include, for example, *Corynebacterium glutamicum, Brevibacterium flavum, Brevibacterium lactofermentum,* and *Flavobacterium rigense.*

L-Arginine producing strains include, for example, *Corynebacterium glutamicum, Brevibacterium flavum, Serratia marcescens, Escherichia coli,* and *Bacillus subtilis.*

L-Alanine producing strains include, for example, *Brevibacterium flavum,* and *Arthrobacter oxydans.*

L-Histidine producing strains include, for example, *Corynebacterium glutamicum, Brevibacterium flavum, Brevibacterium ammoniagenes, Serratia marcescens, Escherichia coli, Bacillus subtilis,* and *Streptomyces coelicolor.*

L-Proline producing strains include, for example, *Corynebacterium glutamicum, Kurthia catenaforma, Serratia marcescens,* and *Escherichia coli.*

L-Phenylalanine producing strains include, for example, *Corynebacterium glutamicum, Brevibacterium flavum, Brevibacterium lactofermentum,* or *Escherichia coli.*

L-Aspartic acid producing strains include, for example, *Brevibacterium flavum, Bacillus megatherium, Escherichia coli,* and *Pseudomonas fluorescens.*

L-Tyrosine producing strains include, for example, *Corynebacterium glutamicum, Brevibacterium flavum, Brevibacterium lactofermentum,* and *Escherichia coli*.

L-Methionine producing strains preferably are *Corynebacterium glutamicum.*

Serine producing strains include, for example, *Corynebacterium glutamicum, Brevibacterium flavum, Brevibacterium lactofermentum,* and *Arthrobacter oxydans.*

L-Serine producing strains include, for example, *Corynebacterium acetoacidophilum,* and *Brevibacterium lactofermentum.*

L-Valine producing strains include, for example, *Brevibacterium lactofermentum, Serratia marcescens,* and *Klebsiella pneumoniae.*

L-Leucine producing strains include, for example, *Corynebacterium glutamicum, Brevibacterium lactofermentum,* and *Serratia marcescens.*

The above-mentioned microorganisms capable of producing L-amino acids may be ones which have been isolated from the natural environment or modified in some properties by mutagenesis or genetic recombination. Examples thereof include, for example, *Flavobacterium rigense,* disclosed in JP 02-219582 A, which has improved productivity of L-threonine, and *Corynebacterium glutamicum,* disclosed in JP 03-500486 W, which has improved productivity of L-alanine.

In the case of producing an L-amino acid by the method for producing a chemical substance by continuous fermentation according to the present invention, separation and purification of the L-amino acid contained in the cultured medium produced can be carried out by combinations of methods, such as concentration, distillation, and crystallization, which are conventionally known.

[Examples]

A hollow fiber membrane module of the present invention and a method for producing a chemical substance by continuous fermentation will be described in more detail by way of Examples and Comparative Examples. However, the present invention is not limited to these Examples.

(Reference Example 1)

Evaluation of Tensile Strength of Sealing Agent before and after Steam Treatment

The evaluation of a tensile strength of a sealing agent was performed by the following method. The evaluation was performed based on JIS K 6251 (2004) using TENSILON (TOYO BALDWIN TMIRTM-100). The specimen of the sealing agent used was a Dumbbell No.3 type specimen defined in JIS K 6251 (2004) in which only a thickness was changed from 2 mm to 1 mm.

With respect to the specimen made of the sealing agent with or without treating with steam at 121°C for 24 hours, the tensile strength was evaluated (n = 5), and then a decrease in tensile strength due to a steam treatment was calculated from the evaluation results of the untreated specimen and the specimen after subjecting to the steam treatment.

$$\text{Decrease [\%] in tensile strength} = (\text{tensile strength [MPa]}$$
$$\text{of untreated specimen} - \text{tensile strength [MPa] of specimen after}$$
$$\text{steam treatment}) / (\text{tensile strength [MPa] of untreated specimen})$$
$$\times 100$$

(Reference Example 2)

Evaluation of Moist Heat Resistance of Sealing Member of Hollow Fiber Membrane Module

Moist heat resistance of a sealing member of a hollow fiber membrane module was evaluated by the following method. The hollow fiber membrane module was treated with steam at 121°C for 24 hours, and then it was visually confirmed whether or not peeling occurs at the bonding portion between a sealing agent and a membrane, and the bonding portion between a sealing agent and housing.

(Reference Example 3)

Production of L-Threonine by Continuous Fermentation

Using a continuous fermenter illustrated in Fig. 4, continuous fermentation of L-threonine was carried out. Operation conditions in continuous fermentation of L-threonine are as follows.

- Microorganisms: Strains of *Providencia rettgeri* SGR588-77 (FERM P-10528)
- Fermentation raw material: L-Threonine fermentation medium (Table 1)

- Fermentation broth volume: 40 L
- Hollow fiber membrane module volume: 0.21 L (inner diameter of 35 mm, length of 220 mm)
- Temperature: 37 (°C)
- Fermenter stirring rate: 200 rpm
- Sterilization: All of a fermenter containing a hollow fiber membrane module, and a fermentation raw material to be used are subjected to high-pressure (2 atm) steam sterilization in an autoclave at 121°C for 20 minutes
- pH Adjustment: pH is adjusted to 7 using an aqueous 28% ammonia solution
- Circulation pump flow rate: 40 L/min
- Filtration rate: 2 L/h (constant)
- Fermenter gas supply amount: 1 L/min

Table 1

| L-Threonine fermentation medium | |
|---|---|
| Components | Concentration |
| Glucose | 100 g/L |
| Yeast Nitrogen base w/o amino acid (Difco) | 6.7 g/L |
| 19 Standard amino acids excluding leucine | 152 mg/L |
| Leucine | 760 mg/L |
| Inositol | 152 mg/L |
| p-Aminobenzoic acid | 16 mg/L |
| Adenine | 40 mg/L |
| Uracil | 152 mg/L |

First, strains of *Providencia rettgeri* SGR588-77 scraped off from an agar medium were inoculated in a test tube containing 5 mL of a glucose bouillon medium (1% glucose, 3% bouillon (manufactured by Nippon Suisan Kaisha, Ltd.)). While stirring at a temperature of 37°C and a rotational speed of 140 rpm, culture was performed (pre-preculture). Next, the pre-prefermentation broth obtained in pre-preculture was inoculated in a 1,000 mL Erlenmeyer flask containing 200 mL of a glucose bouillon medium charged therein, and culture was performed while stirring at a temperature of 37°C and a rotational speed of 140 rpm (preculture). The obtained prefermentation broth was inoculated in a continuous fermenter containing 40 L of a L-threonine fermentation medium (Table 1) charged therein illustrated in Fig. 4, and then cuture was performed for 24 hours. Immediately after culture, filtration of the fermentation broth by the hollow fiber membrane module and continuous supply of a L-threonine fermentation medium were performed, and then the production of L-threonine by continuous fermentation was performed by continuously culturing while performing medium supply control so as to make the amount of the fermentation broth of the continuous fermenter constant. Continuous fermentation was carried out for 500 hours. During continuous fermentation, transmembrane pressure differences at the supply and permeation sides of the membrane were measured, and then a transmembrane pressure difference increase rate was determined from the transmembrane pressure difference after a lapse of 50 hours from the beginning of filtration and the transmembrane pressure difference after a lapse of 500 hours.

Transmembrane pressure difference increase rate [kPa/h]

= (transmembrane pressure difference [kPa] after a lapse of 500 hours − transmembrane pressure difference [kPa] after a lapse of 50 hours)/(500 [h] − 50 [h]).

(Example 1)

Twenty (20) parts by weight of polyethersulfone (Victrex 200), 10 parts by weight of polyvinylpyrrolidone (having a weight average molecular weight 360,000), 65 parts by weight of N-methyl-2-pyrrolidone and 5 parts by weight of isopropanol were mixed and dissolved, followed by degassing while being left to stand to give a raw liquid for membrane production. A mixed solution of this raw liquid for membrane production and N-methyl-2-pyrrolidone/$H_2O$ (= 80/20 (wt/wt)) as a hollow section-forming fluid was discharged from a double pipe spinneret and solidified by passing through a water bath at 20°C disposed 30 mm below the spinneret, followed by washing with water to obtain a hollow fiber membrane. The membrane washed with water was wound on a cassette.

Next, the hollow fiber membrane thus produced was immersed in ethanol and then immersed in hexane to dehydrate the hollow fiber membrane. Thereafter, polyvinylpyrrolidone was cross-linked by subjecting to a heat treatment under an atmosphere at 150°C for 2 to 30 hours. The obtained hollow fiber membrane had an outer diameter of 1,300 $\mu$m, an inner diameter of 810 $\mu$m and a membrane surface average pore diameter of 100 nm.

Using the above-mentioned hollow fiber membrane, a hollow fiber membrane module was produced. A molded article of a polysulfone resin was used as a module housing. The thus produced membrane filtration module had a volume of 0. 21 L, and an effective filtration area of the membrane filtration module was 2, 500 cm$^2$. An epoxy resin (main component: bisphenol F type epoxy resin, curing agent: aliphatic polyamine) was used as a sealing agent of the module. Filling of the sealing agent was carried out by a centrifugal potting method to produce a hollow fiber membrane module of an aspect illustrated in Fig. 1.

The above-mentioned sealing agent was evaluated by the method illustrated in Reference Example 1. As a result, a decrease in tensile strength after the steam treatment was 2.0%. The above-mentioned hollow fiber membrane module was evaluated by the method illustrated in Reference Example 2. As a result, peeling was not recognized in the sealing member.

Using the above-mentioned hollow fiber membrane module, continuous fermentation illustrated in Reference Example 3 was carried out. As a result, an increase rate of a transmembrane pressure difference was 0.21 × 10$^{-3}$ kPa/h.

(Example 2)

In the same manner as in Example 1, except that an epoxy resin (main component: bisphenol A, curing agent: aliphatic polyamine) was used as the sealing agent of the module, a hollow fiber membrane module was produced.

The sealing agent was evaluated by the method illustrated in Reference Example 1. As a result, a decrease in tensile strength after the steam treatment was 4.0%. The above-mentioned hollow fiber membrane module was evaluated by the method illustrated in Reference Example 2. As a result, peeling was not recognized in the sealing member.

Using the above-mentioned hollow fiber membrane module, continuous fermentation illustrated in Reference Example 3 was carried out. As a result, an increase rate of a transmembrane pressure difference was 0.21 × 10$^{-3}$ kPa/h.

(Example 3)

In the same manner as in Example 1, except that a urethane resin (isocyanate: Polymeric MDI., polyol: polybutadiene based polyol) was used as the sealing agent of the module, a hollow fiber membrane module was produced.

The sealing agent was evaluated by the method illustrated in Reference Example 1. As a result, a decrease in tensile strength after the steam treatment was 19.4%. The above-mentioned hollow fiber membrane module was evaluated by the method illustrated in Reference Example 2. As a result, peeling was not recognized in the sealing member.

Using the above-mentioned hollow fiber membrane module, continuous fermentation illustrated in Reference Example 3 was carried out. As a result, an increase rate of a transmembrane pressure difference was 0.23 × 10$^{-3}$ kPa/h.

(Example 4)

To 102 parts by weight of dimethylacetamide (DMAc), 30 parts by weight of polyvinylpyrrolidone (K-30, manufactured by NACALAI TESQUE, INC.) and 27 parts by weight of a polysulfone resin (Udel (regsitered trademark) P-3500: PS, manufactured by Solvay Advanced Polymers Inc.) were added and dissolved at 60°C for 5 hours, followed by degassing while being left to stand to give a raw liquid for membrane production.

A mixed solution of this raw liquid for membrane production and DMAc/$H_2O$ (= 95/5 (wt/wt)) as a hollow section-forming fluid was discharged from a double pipe spinneret and solidified by passing through a water bath at 60°C disposed 30 mm below the spinneret, followed by washing with water to obtain a hollow fiber membrane. The membrane washed with water was wound on a cassette.

Thereafter, an ethylene-vinyl alcohol copolymer resin Soarnol (regsitered trademark) D2908 (ethylene content 29 mol%) manufactured by The Nippon Synthetic Chemical Industry Co. , Ltd. was dissolved in an aqueous 50% by weight isopropyl alcohol solution to prepare a solution in which the concentration of an ethylene-vinyl alcohol copolymer solution is 0.5% by weight. The above-mentioned hollow fiber membrane was immersed in the ethylene-vinyl alcohol copolymer solution maintained at 60°C for 10 minutes and then dried to obtain a hollow fiber membrane in which a coating weight of an ethylene-vinyl alcohol copolymer is 1.3% by weight. The obtained hollow fiber membrane had an outer diameter of 1,390 $\mu$m, an inner diameter of 810 $\mu$m and a membrane surface average pore diameter of 50 nm.

In the same manner as in Example 1, except that the above-mentioned hollow fiber membrane was used, and a urethane resin (isocyanate: Polymeric MDI., polyol: dimer acid modified polyol + polybutadiene based polyol (prepared by mixing two types of polyols in a ratio of OH value 1:1)) was used as the sealing agent of the module, a hollow fiber membrane module was produced.

The above-mentioned sealing agent was evaluated by the method illustrated in Reference Example 1. As a result, a decrease in tensile strength after the steam treatment was 1.0%. The above-mentioned hollow fiber membrane module was evaluated by the method illustrated in Reference Example 2. As a result, peeling was not recognized in the sealing member.

Using the above-mentioned hollow fiber membrane module, continuous fermentation illustrated in Reference Example 3 was carried out. As a result, an increase rate of a transmembrane pressure difference was $0.15 \times 10^{-3}$ kPa/h.

(Comparative Example 1)

In the same manner as in Example 1, except that a urethane resin (isocyanate: carbodiimide modified isocyanate, polyol: castor oil based polyol) was used as the sealing agent of the module, a hollow fiber membrane module was produced.

The sealing agent was evaluated by the method illustrated in Reference Example 1. As a result, a decrease in tensile strength after the steam treatment was 39.5%. The above-mentioned hollow fiber membrane module was evaluated by the method illustrated in Reference Example 2. As a result, peeling was recognized in the space between a housing and a sealing agent. The above-mentioned hollow fiber membrane module was not used in continuous fermentation since bacterial contamination may be generated from the peeled portion.

(Comparative Example 2)

Eighteen (18) parts by weight of polyvinylidene difluoride having a weight average molecular weight of 417,000 was mixed with 82 parts by weight of N-methyl-2-pyrrolidone, and then the mixture was dissolved at a temperature of 140°C. This polymer solution and an aqueous 40% by weight N-methyl-2-pyrrolidone solution as a hollow section-forming fluid were discharged from a double pipe spinneret and then solidified in a water bath at a temperature of 20°C to produce a hollow fiber membrane. The obtained hollow fiber membrane had an outer diameter of 1,260 $\mu$m, an inner diameter of 780 $\mu$m and a membrane surface average pore diameter of 120 nm.

Using this hollow fiber membrane, a hollow fiber membrane module was produced in the same manner as in Example 3. The hollow fiber membrane module was evaluated by the method illustrated in Reference Example 2. As a result, peeling was recognized in the space between a hollow fiber membrane and a sealing agent in 3% of the total. The above-mentioned hollow fiber membrane module was not used in continuous fermentation since bacterial contamination may be generated from the peeled portion.

Table 2

| | Example 1 | Example 2 | Example 3 | Example 4 | Comparative Example 1 | Comparative Example 2 |
|---|---|---|---|---|---|---|
| Membrane material | Polyethersulfone | Polyethersulfone | Polyethersulfone | Polysulfone | Polyethersulfone | Polyvinylidene difluoride |
| Membrane surface average pore diameter (nm) | 100 | 100 | 100 | 50 | 100 | 120 |
| Pure water permeability coefficient ($m^3/m^2/h/50kPa$) | 1.3 | 1.3 | 1.3 | 1.1 | 1.3 | 0.8 |
| Application amount (% by weight) of ethylene-vinyl alcohol copolymer | 0 | 0 | 0 | 1.3 | 0 | 0 |
| Sealing agent material | Epoxy resin | Epoxy resin | Polyurethane resin | Polyurethane resin | Polyurethane resin | Polyurethane resin |
| Decrease (%) in tensile strength of sealing agent due to steam treatment | 2.00% | 4.00% | 19.40% | 1.00% | 39.50% | 19.40% |
| Peeling occurred between housing and sealing agent | None | None | None | None | Peeling occurred | None |
| Peeling occurred between hollow fiber membrane and sealing agent | None | None | None | None | None | Peeling occurred in 3% of the total |
| Transmembrane pressure difference increase rate (kPa/h) | $0.21 \times 10^{-3}$ | $0.21 \times 10^{-3}$ | $0.23 \times 10^{-3}$ | $0.15 \times 10^{-3}$ | - | - |

[Industrial Applicability]

Since the hollow fiber membrane module of the present invention has sufficient durability against steam sterilization and can maintain stable filtration property over a long period of time, the hollow fiber membrane module is widely used in the fermentation industry, and thus making it possible to stably produce a chemical substance, which is a fermentation product, at low cost.

[Reference Signs List]

| | |
|---|---|
| 1: | Hollow fiber membrane module |
| 2: | Hollow fiber membrane |
| 3: | Housing |
| 4: | Hollow fiber membrane sealing member |
| 5: | Small bundle sealing member |
| 6: | Upper cap |
| 7: | Lower cap |
| 8: | Fermentation broth inlet |
| 9: | Filtrate outlet |
| 10: | Fermentation broth outlet |
| 11: | Tubular case |
| 12: | O-ring |
| 13: | Fermenter |
| 14: | Separation membrane module |
| 15: | Temperature controller |
| 16: | Stirrer |
| 17: | pH Sensor/controller |
| 18: | Level sensor/controller |
| 19: | Fermenter gas supply device |
| 20: | Circulation pump |
| 21: | Fermentation raw material supply pump |
| 22: | Neutralizer supply pump |
| 23: | Circulation return control valve |
| 24: | Filtration pump |
| 25: | Filtrate reservoir |
| 26: | Fermentation raw material tank |
| 27: | Neutralizer tank |

**Claims**

1. A hollow fiber membrane module comprising a housing and a hollow fiber membrane filled in the housing, the space between the hollow fiber membrane and the housing being sealed with a sealing agent; wherein the hollow fiber membrane is made of a polysulfone based resin; a membrane surface average pore diameter is 5 nm or more and 500 nm or less; a pure water permeability coefficient under 50 kPa at 25°C is 0.05 $m^3/m^2$/hour or more and 5 $m^3/m^2$/hour or less; the sealing agent is at least one selected from a polyurethane resin and an epoxy resin; and a decrease in tensile strength after bringing the sealing agent into contact with saturated steam at 121°C for 24 hours is 25% or less.

2. The hollow fiber membrane module according to claim 1, wherein a tubular case and a hollow fiber membrane are filled in a housing; the space between the hollow fiber membrane and the tubular case is sealed with a sealing agent; and the space between the tubular case and the housing is fluid-tightly fixed by a gasket.

3. The hollow fiber membrane module according to claim 1 or 2, wherein a polyol component of the polyurethane resin used in the sealing agent includes at least one selected from the group consisting of a polybutadiene based polyol, a dimer acid modified polyol, an epoxy resin modified polyol and a polytetramethylene glycol.

4. The hollow fiber membrane module according to claim 1 or 2, wherein the epoxy resin used in the sealing agent includes at least one selected from the group consisting of a bisphenol type epoxy resin, a novolak type epoxy resin, a naphthalene type epoxy resin and a cyclopentadiene type epoxy resin.

5. The hollow fiber membrane module according to any one of claims 1 to 4, wherein 0.1% by weight or more and 10.0% by weight or less of an ethylene-vinyl alcohol copolymer is retained on a surface of the hollow fiber membrane.

6. A method for producing a chemical substance, which comprises the steps of using a fermentation broth containing a fermentation raw material, a chemical substance, and microorganisms or cultured cells, filtering the fermentation broth through a separation membrane module, collecting the chemical substance from the filtrate, and at the same time retaining or circulating the unfiltered liquid in the fermentation broth; and adding the fermentation raw material to the fermentation broth; the chemical substance being produced by continuous fermentation; wherein the hollow fiber membrane module according to any one of claims 1 to 5 is used as the separation membrane module.

## Fig. 1

Fig. 2

Fig. 3

Fig. 4

| | INTERNATIONAL SEARCH REPORT | International application No. |
|---|---|---|
| | | PCT/JP2011/079831 |

**A. CLASSIFICATION OF SUBJECT MATTER**
*B01D71/68*(2006.01)i, *B01D61/14*(2006.01)i, *B01D63/00*(2006.01)i, *B01D63/02* (2006.01)i, *C12P1/00*(2006.01)i

According to International Patent Classification (IPC) or to both national classification and IPC

**B. FIELDS SEARCHED**

Minimum documentation searched (classification system followed by classification symbols)
B01D71/68, B01D61/14, B01D63/00, B01D63/02, C12P1/00

Documentation searched other than minimum documentation to the extent that such documents are included in the fields searched
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2010-29108 A (Toray Industries, Inc.), 12 February 2010 (12.02.2010), claim 1; paragraphs [0037], [0050], [0097] (Family: none) | 1-6 |
| Y | JP 2008-237213 A (Toray Industries, Inc.), 09 October 2008 (09.10.2008), paragraph [0024] (Family: none) | 1-6 |
| Y | JP 2010-167372 A (Toray Industries, Inc.), 05 August 2010 (05.08.2010), paragraph [0002]; fig. 1 (Family: none) | 2 |

☒ Further documents are listed in the continuation of Box C.　　☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 09 March, 2012 (09.03.12) | 19 March, 2012 (19.03.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2011/079831

C (Continuation).　DOCUMENTS CONSIDERED TO BE RELEVANT

| Category* | Citation of document, with indication, where appropriate, of the relevant passages | Relevant to claim No. |
|---|---|---|
| Y | JP 6-170193 A　(Asahi Chemical Industry Co., Ltd.),<br>21 June 1994 (21.06.1994),<br>claims<br>(Family: none) | 5 |
| A | JP 2-107318 A　(Daicel Chemical Industries, Ltd.),<br>19 April 1990 (19.04.1990),<br>entire text<br>(Family: none) | 1-6 |
| A | JP 8-71378 A　(Kaneka Corp.),<br>19 March 1996 (19.03.1996),<br>entire text<br>(Family: none) | 1-6 |
| P,X | WO 2011/058983 A1　(Toray Industries, Inc.),<br>19 May 2011 (19.05.2011),<br>claims; paragraphs [0011], [0027], [0037], [0059], [0071]<br>(Family: none) | 1,3,4,6 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005333886 A
- WO 2011058983 A
- JP 2008237213 A
- JP 2219582 A
- JP 3500486 W

**Non-patent literature cited in the description**

- Kagaku Binran Kisohen. Maruzen Co., Ltd, 1984, II-82